Europäisches Patentamt

European Patent Office

Office européen des brevets

(19)

(11) Publication number: **0 074 231**
**A2**

(12) **EUROPEAN PATENT APPLICATION**

(21) Application number: **82304538.0**

(22) Date of filing: **27.08.82**

(51) Int. Cl.³: **A 61 B 5/10**

(30) Priority: **27.08.81 AR 286564**

(43) Date of publication of application:
**16.03.83 Bulletin 83/11**

(84) Designated Contracting States:
**AT BE CH DE FR GB IT LI LU NL SE**

(71) Applicant: **Jonch, Ernesto Osvaldo**
**Tacuari 791**
**Buenos Aires 1071(AR)**

(72) Inventor: **Jonch, Ernesto Osvaldo**
**Tacuari 791**
**Buenos Aires 1071(AR)**

(74) Representative: **Harvey, David G. et al,**
**Graham Watt & Co. Riverhead**
**Sevenoaks Kent TN13 2BN(GB)**

(54) Apparatus for dynamic podologic studies.

(57) An apparatus for carrying out dynamic podologic study capable of allowing the viewing and recording of images is given whereby said device includes a frame (1) supporting a transparent and preferably horizontal track (2) having sufficient size so that a patient may take several normal steps thereon, and supporting in turn a mirror (5) located in a plane below said track, and including rails (3, 4) for a displaceable support (6) which holds at least one camera (11) with its focus directed preferably towards said mirror (5).

FIG.1.

EP 0 074 231 A2

- 1 -

## APPARATUS FOR DYNAMIC PODOLOGIC STUDIES

### Summary of the Invention

The instant invention relates to a device for carrying out dynamic podologic studies. Podology is the scientific study of the morphology and physiology of the feet. Although this invention may be used for a plurality of applications, it is particularly useful for orthopedic studies including diagnosis and analysis of podologic support, gait, and compensating vices, as well as mechanical pathologies and many other similar studies.

The present invention relates to devices consisting of a combination of means mutually interrelated to facilitate registration and study of the techniques of walking and support by a patient, said means capable of reproducing said data on video screens and being capable of being stopped, played back, and repeated in order that the medical or other specialist may have the opportunity to gather all information and data needed to perform improved diagnosis and suggest treatments based on techniques improved over those that are known.

## Brief Description of the Invention

The means and apparatus of the instant invention permits analysis and study of a higher quality than that available from the present techniques. This is particularly true since the ability to stop the image on the screen at any time may be effected even in the intermediate positions of the patient's activity, in a stage in which the patient can not ordinarily be able to stop. The proper study of all movements, deformations, and muscle reactions, among other acts at any moment during gait, may give valuable information for the diagnosis and the ability to stop action by video techniques at any moment, contribute to the efficiency of such analysis and diagnosis.

The unique results are a direct consequence of the special combination of apparatus or means of this invention which generally consists of a transparent track on which the patient may walk upon in a normal way, a frame supporting said track and supporting a plane mirror located below said track so that it reflects the images of the feet during movement including the gait, so that they may be viewed by . at least one television camera directed towards the support zone of the feet and held by a support that in turn displaces the camera at the same speed and direction than said gait of the patient, said camera being of the type useful for recording.

Images may be obtained by conventional film but it is preferred that video tape in a conventional cassette for reproduction be employed for use in video monitors.

The mirror located below the track extends horizontally, but in one of the embodiments of this invention it may have a slope towards a side or may be on a plane parallel to that of the track, according to the alternatives related to the location of a single camera. In fact, said camera may be located close to the track or thereon. In the first case, the longitudinal mirror is inclined towards the side in which the camera is located, while in the second case the mirror will be horizontal to both directions, that is both longitudinal and transverse.

The above features correspond to an embodiment where the track is horizontal, since if the track has an upward or downward slope, the mirror will have the same longitudinal direction, but may be inclined with respect to the plane of the track.

This invention encompasses alternative embodiments for displacing the cameras in the same direction and at the same speed of the patient's movement gait.

To this end, the support holding any of the cameras will be mounted, preferably on bearings, and will be capable

of providing said displacement as actuated by the patient, the observer, or by any other convenient technique. The more practical method is generally where the patient provides the movement or displacement, for example by means of a handle connected to said support and at the control of the patient.

The most convenient location of the camera is that in which the camera may simultaneously take the lower part of the legs with the feet during movement or gait, along with the images of the feet soles as reflected by the mirror so that in the image obtained includes the upper and lower part of the feet simultaneously at any time during movement.

Alternatively, when employing more than one video camera from different observation points, images can be obtained from different angles which improve the observation and thus the studies, since in these images various simultaneous aspects may be verified and compared which may not be perfectly clear with a single viewing device.

Therefore, this invention includes embodiments with a greater amount of viewing alternatives than previously known by a system which allows for viewing and recording by the use of television cameras, mutually independent, and directed in a way that in some of them feet support images are taken reflected on the plane mirror, and in the others images simultaneous to said direct images may be obtained, lateral
or dorsal, from one side or from the other, thus recording
in a greater detail all muscle reactions, positions adopted
by the feet during gait, sole side and upper deformations,
etc. All these data being obtained simultaneously, so that
the recording and reproducing on independent monitors will
permit studies with a maximum degree of information, thus
improving the possibility of a proper and safe diagnosis.

In accordance with a preferred embodiment of the
invention, the support displaceable on the transparent track
is provided with holding means for three television cameras
more than in the embodiment in which a single camera is
included and, besides, in the end portion of the track where
the patient starts his gait, another similar element is
included, preferably provided with a distance regulator or
automatic zoom, capable of following the patient forward
gait, always taking the support of his soles reflected by
the mirror.

Under the cited conditions, the displaceable support
will be provided with a plurality of television or other
type of camera. In one embodiment the support will be
provided with four television cameras, one at each side
looking at the dorsal portions of the patient's feet, a
third camera taking the frong and upper portion of both dorsal parts simultaneously and the fourth camera taking the sole planes of both feet refelcted by the mirror. In turn, the camera affixed to the end of the track, i.e. the fifth camera, will take from the rear part said sole planes reflected by the mirror.

Optionally, the same support may also hold lighting means properly directed in order to attain a clear image of all details during the gait of the patient.

In order to obtain the mentioned advantages, to which users and those skilled in the art may add many others, and to facilitate the understanding of the constructive, constructive and functional characteristics of the instant invention, some prepferred embodiments of the invention will be described as follows, illustrated schematically and without a determined scale. These examples are not to be considered as limiting the scope of the invention, but rather as simply illustrating and explaining the basic concepts of the invention.

Brief Description of the Drawings

For a better understanding of the invention as well as other objects and further features thereof, reference is made to the following detailed descriptions of embodiments of this invention, some of which are preferred. The above disclosure is hereby supplemented by the following descriptions which when taken with the drawings more clearly demonstrate the various aspects of this invention.

In Figure 1, a perspective view of an apparatus or device according to the present invention is given showing one embodiment employing a single camera which may be a video camera of conventional type located at the side of the path of movement of the patient;

In Figure 2, a side view of the apparatus or device is given in which a single camera is located on the path of movement and in front of the patient;

In Figure 3, another perspective view is given in which the embodiment shown includes a plurality of cameras;

In Figure 4, a front view of generally the same device as shown in the embodiment of Figure 3 is given; and

In Figure 5, a side view of the device of Figure 3 is shown.

Throughout the drawings in all figures the same reference numerals correspond to the same or substantially the same, or equivalent parts or constituting elements of the assemblies selected for detailing the embodiments of this invention.

## Detailed Description of the Invention

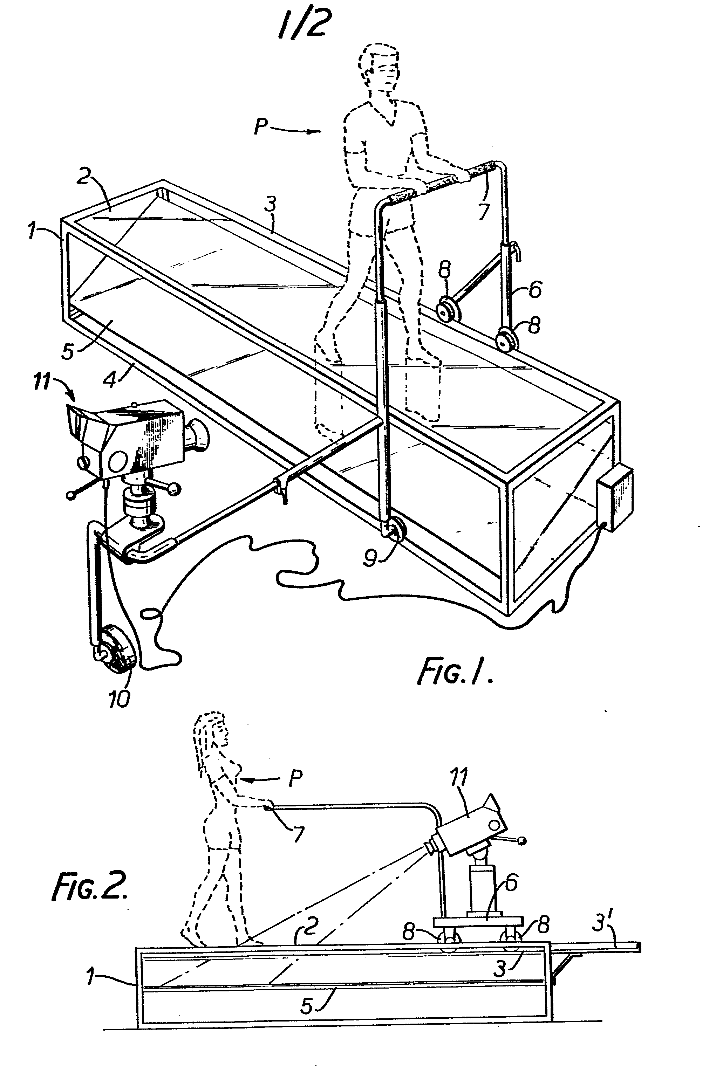

Referring now to Figure 1, the embodiment includes a frame 1 which holds a track 2 which may be made of transparent material such as glass, plastic, crystal or the like, also including upper rails generally shown as 3 and lower rails generally shown as 4, each horizontal and generally but not necessarily parallel. The frame 1 and rails 3 and 4 provide support for inclined reflecting surface or mirror 5. A movable or displaceable support 6 provided with a hand rail 7 and is supported by wheels 8 and 9 on the rails 3 and 4, and may alternatively have an outer wheel 10 positioned on the floor in order to support a holding assembly for a conventional and appropriate filming or video or other type of camera assembly 11 which may be directed towards the zone in which the feet and feet images are projected onto the mirror 5. In this embodiment, a patient may walk on the transparent track 2 and in turn push the camera by pushing rail 7 in order that said camera 11 may record the gait which can be projected on a monitor in order that the specialist may study the details.

In the embodiment shown in Figure 2, the camera 11 is located in front of the patient P and thus the mirror 5 is located at a plane lower and parallel to that of track 2. Here the camera support rails will be the upper rails and may have sufficient projections 3' to provide enough distance between the camera 11 and the patient to allow the complete track area to be used for study.

Within the scope of this invention, as has already been stated, alternatives with mechanical devices of any type may be included, in order that the movements of the camera may be carried out simultaneously with those of the patient, thus allowing the various embodiments intended within the scope of this invention.

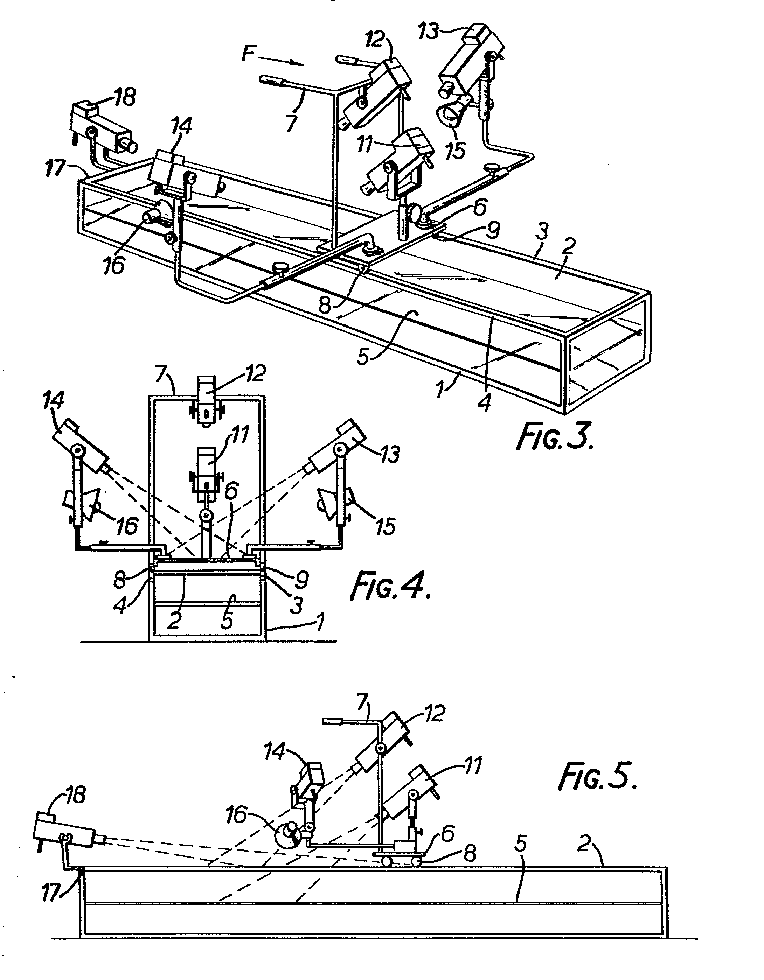

The embodiments illustrated in Figures 3, 4, and 5 have frame 1 supporting horizontal track 2 of transparent material such as glass or plastic or crystal or the like, and including railing means or rails 3 and 4 also horizontal and mutually parallel, and also supporting a plane mirror 5 which will be preferably horizontal, although it may also have an upward slope in the direction of the patient's gait as indicated by arrow F, the movable or displaceable support 6 being provided with a handle rail 7 and mounted on wheels 8 and 9 positioned on said rails 3 and 4 which may be supplemented by other similar rear wheels in order to maintain the horizontal condition of said support 6.

An alternative included in this embodiment consists of support 6 holding four cameras indicated with reference numerals 11, 12, 13, and 14, held by respective arms which, preferably, will be adjustable so that each camera may be correctly focused. The arm configurations shown in the figures may be replaced by equivalent structures, since this fact does not affect the novelty of the invention.

One of the front cameras 11 or 12 is focused for recording sole images reflected by the mirror 5, while the other is directed in order to record dorsal and front parts of both feet in a direct manner. In turn, the two side cameras 13 and 14 are focused in order to take simultaneously the outer or inner dorsal parts of both feet.

Optionally, support 6 may also hold lighting reflectors such as those illustrated with reference numerals 15 and 16, in order to ensure permanent uniform lighting on the feet while the patient is walking.

Positioned at an end portion of track 2 as shown as 17 in Figure 5, the frame may be modified to hold another or fifth camera 18 which, since it is fixed without accompanying the movable or displaceable support 6 during the gait of the patient, will allow for fixed study of patient movement and may be provided with a focusing distance regulator or other type of device such as a so-called "zoom" lens which may have an automatic driving means which operates as a function of the advance of the support 6.

When simultaneous images are taken with simultaneous cameras, recordings will be obtained which may be simultaneously reproduced in respective monitors so that the physician may have ample information which will allow him to make the diagnosis and determine the type of treatment to be given or applied to the patient. The physician may by this invention select any number of viewing points and the number of cameras to be employed is not critical to the invention.

When employing the above described devices and apparatus for carrying out dynamic podologic investigations, modifications and/or alternatives may be introduced all of which should be considered as embodiments comprised within the scope of the present invention, which invention is only to be limited by the scope of the following claims.

WHAT IS CLAIMED IS:

1. An apparatus for permitting dynamic podologic study capable of allowing and recording the gait of a patient in motion comprising a frame supporting a transpatent track having a strength and width sufficient to support a patient and a length sufficient to allow a patient at least one normal step thereon, said frame supporting a reflecting surface whereby the sole of the patient in motion may be viewed simultaneous with view of the normal position located below said track, and includings means for movement of at least one viewing camera capable for recording the gait images of said patient.

2. The device of Claim 1 wherein said reflecting surface is transversely inclined with respect to the greater dimension of the track and the camera is positioned at a side thereof.

3. The device of Claim 1 wherein said reflecting surface is parallel to the plane of said track and the camera is located above the plane of said track and is displaced forwardly with respect to the direction of movement of said patient.

4. The device of Claim 1 wherein the support holding said camera has an extension handle being position to be movable by the patient during movement by the patient on said track.

5. The device of Claim 1 to 4, wherein the support is movable along the length of said track.

6. The device of Claim 1 wherein one end of the track corresponding to the start of the patient's gait and independently from said moveable support, and a second camera directed to the rear protion of the feet of said patient, and another camera focused towards the dorsal protion of said feet.

7. The device of Claim 6 wherein the camera located on the end of the track includes a distance focusing regulating means automatically governed according to the advance of the movable and displaceable support.

8. The davice of Claim 6 wherein the movable or displaceable support holds a plurality of cameras positioned to view at least the directed to the dorsal portions of the patient's feet and the sole portions thereof.

9. The device of Claims 6 to 8 wherein said cameras are positioned and regulated to be adjusted for height and direction.

10.    The device as claimed in Claims 6 to 9 wherein the displaceable support also holds lighting means at least sidewards oriented towards the patient's feet and displaceable with said support.

*Fig.1.*

*Fig.2.*

Fig.3.

Fig.4.

Fig.5.